(19) Europäisches Patentamt
European Patent Office
Office européen des brevets

(11) **EP 1 572 782 B1**

(12) **EUROPÄISCHE PATENTSCHRIFT**

(45) Veröffentlichungstag und Bekanntmachung des Hinweises auf die Patenterteilung:
**25.02.2015   Patentblatt 2015/09**

(21) Anmeldenummer: **03758060.2**

(22) Anmeldetag: **24.10.2003**

(51) Int Cl.:
*A61L 15/60* (2006.01)    *B29B 7/00* (2006.01)
*C08F 8/00* (2006.01)

(86) Internationale Anmeldenummer:
**PCT/EP2003/011830**

(87) Internationale Veröffentlichungsnummer:
**WO 2004/037900 (06.05.2004 Gazette 2004/19)**

(54) **ZWEISTUFIGES MISCHVERFAHREN ZUR HERSTELLUNG   EINES ABSORBIERENDEN POLYMERS**

TWO-STAGE MIXING METHOD FOR PRODUCING AN ABSORBENT POLYMER

PROCEDE DE MELANGE EN DEUX ETAPES POUR PREPARER UN POLYMERE ABSORBANT

(84) Benannte Vertragsstaaten:
**AT BE BG CH CY CZ DE DK EE ES FI FR GB GR HU IE IT LI LU MC NL PT RO SE SI SK TR**

(30) Priorität: **25.10.2002   DE 10249822**

(43) Veröffentlichungstag der Anmeldung:
**14.09.2005   Patentblatt 2005/37**

(73) Patentinhaber: **Evonik Degussa GmbH**
**45128 Essen (DE)**

(72) Erfinder:
• **VAN STIPHOUDT, Manfred**
**47906 Kempen (DE)**
• **VORHOLT, Herbert**
**45721 Haltern (DE)**
• **REIMANN, Armin**
**47877 Willich (DE)**

(74) Vertreter: **Lang, Arne et al**
**Evonik Degussa GmbH**
**DG-IPM-PAT**
**Bau 1042 / PB 15**
**Paul-Baumann-Strasse 1**
**45764 Marl (DE)**

(56) Entgegenhaltungen:
**WO-A-98/49221    US-A- 5 002 986**

**Beschreibung**

Die Erfindung betrifft ein Verfahren zur Herstellung eines absorbierenden Polymers.

Für eine Bildung von sogenannten "superabsorbierenden" Polymeren ist eine Polymerisation verschiedener Arten von normalerweise wasserlöslichen Monomeren, oft jedoch auch von wasserunlöslichen Comonomeren zusammen mit einer Präsenz von Vernetzern erforderlich. Die Zugabe der Vernetzer erfolgt während oder nach der Polymerisation. Derartig superabsorbierende Polymere sind schwachvernetzte, wasserunlösliche Hydrogelpolymere, die im trockenen und im wesentlichen wasserfreien Zustand eine große Fähigkeit zur Wasserabsorption aufweisen. Die aufgenommene Wassermenge kann ein Mehrfaches des Eigengewichts der superabsorbierenden Polymere ausmachen.

Aufgrund dieser hohen Absorptionsfähigkeit eignen sich superabsorbierende Polymere zu wasserabsorbierenden Strukturen und Gegenständen, wie z. B. Babywindeln, Inkontinenzprodukten oder Damenbinden.

Wenngleich das Verhältnis vom aufnehmbaren Wassergewicht zum Trockengewicht der Polymere, i.e. die Absorptionsfähigkeit, für derartige Anwendungen ausreichend ist, ist die Geschwindigkeit, mit der das Wasser aufgenommen wird, d.h. die Absorptionsgeschwindigkeit, begrenzt und für viele Anwendungsfälle unbefriedigend. In vielen Fällen kommt die hohe Absorptionsfähigkeit der superabsorbierenden Polymere gar nicht zum Tragen, da die langsame Absorptionsgeschwindigkeit eine ausreichend schnelle Aufnahme des Wassers verhindert und somit den Einsatz der Polymere in Hygieneanwendungen erschwert.

Die WO-A-98/49221 beschreibt ein Verfahren zur Verbesserung der Eigenschaften absorbierender Polymerteilchen, bei dem die Polymerteilchen, die zuvor beispielsweise im Rahmen einer Nachvernetzungsreaktion für mindestens 10 Minuten bei mindestens 170°C erhitzt worden sind, mit einer wässrigen Additivlösung vermischt werden. Bei dem Vermischen der Polymere mit einer wässrigen Lösung kommt es jedoch zur Agglomeration der Polymerteilchen, die gemäß der Lehre der Druckschrift WO-A-98/49221 nur durch den Zusatz von Additiven beispielsweise in Form von ein- oder mehrwertigen Metallkationen in der wässrigen Lösung verhindert werden kann.

Allgemein liegt der vorliegenden Erfindung die Aufgabe zu Grunde, die sich aus dem Stand der Technik ergebenden Nachteile zu überwinden.

Es ist insbesondere eine Aufgabe der vorliegenden Erfindung, ein Verfahren anzugeben, nach dem superabsorbierende Polymere hergestellt werden können, die eine erhöhte Absorptionsgeschwindigkeit für Wasser aufweisen ohne nennenswert in ihrer Absorptionsfähigkeit nachzulassen. Außerdem sollte dieses Verfahren in einfacher Weise durchzuführen sein und ohne den Einsatz von Additiven, insbesondere ohne den Einsatz organischer Additive auskommen.

Eine weitere Aufgabe der Erfindung besteht vornehmlich darin, ein superabsorbierendes Polymer, Verbunde, die derartige superabsorbierende Polymere enthalten, und chemische Produkte, die derartige superabsorbierende Polymere oder Verbunde enthalten, anzugeben, wobei die superabsorbierende Polymere eine erhöhte Absorptionsgeschwindigkeit für Wasser aufweisen. Außerdem sollten die Absorptionseigenschaften dieser Polymere bei mechanischen Belastungen, wie sie insbesondere beim Transport der Polymerpartikel in Förderanlagen auftreten, nicht nachteilig beeinflusst werden. Insbesondere soll die Absorptionsfähigkeit der Polymere unter einer Druckbelastung durch eine mechanische Belastung möglicht nicht oder allenfalls nur gering vermindert werden (=mechanische Stabilität).

Diese Aufgaben werden gelöst durch den Gegenstand der kategoriebildenden Ansprüche. Vorteilhafte Ausgestaltungen und Weiterbildungen, die einzeln oder in Kombination auftreten können, sind Gegenstand der jeweils abhängigen Ansprüche.

Bei dem erfindungsgemäßen Verfahren zur Herstellung eines absorbierenden Polymers aufweisend einen ersten Mischvorgang, bei dem eine Vielzahl von absorbierenden Polymerteilchen mit einer Flüssigkeit vermischt werden, und einen zweiten Mischvorgang, bei dem die Flüssigkeit im Inneren der Polymerteilchen verteilt, vorzugsweise homogenisiert, wird, werden die Polymerteilchen in dem ersten Mischvorgang mit einer Geschwindigkeit gemischt, dass die Bewegungsenergie der einzelnen Polymerteilchen im Mittel größer ist als die Haftungsenergie zwischen den einzelnen Polymerteilchen, und die Polymerteilchen in dem zweiten Mischvorgang werden mit einer geringeren Geschwindigkeit als im ersten Mischvorgang durchmischt.

In dem ersten Mischvorgang werden die Polymerteilchen an ihrer Oberfläche mit der Flüssigkeit benetzt. Die Polymerteilchen werden beim ersten Mischvorgang mit einer höheren Geschwindigkeit als beim zweiten Mischvorgang durchmischt.

Die hohe Geschwindigkeit bewirkt eine Fluidisierung der Polymerteilchen, welche verhindert, dass die Polymerteilchen aneinander haften bleiben und verklumpen. Die hohe Geschwindigkeit führt dazu, dass im Falle einer Aneinanderhaftung zweier Polymerteilchen bei einem Aufprall eines dritten Polymerteilchens bzw. beim Aufprall der verklumpten Polymerteilchen auf eine Wand z.B. des Mischers sich die beiden verklumpten Polymerteilchen voneinander lösen.

Die Bedingung, dass die Bewegungsenergie der einzelnen Polymerteilchen im Mittel größer als die Haftungsenergie zwischen den einzelnen Polymerteilchen ist, hat zur Folge, dass die Polymerteilchen vorwiegend als Individuen und nicht als Verbund, Agglomeration oder Tropfen vorzufinden sind. Da die Haftungsenergie kleiner ist als die Bewegungsenergie, befinden sich die Polymerteilchen in stetiger Bewegung als Individuen.

Durch die Bewegung der Polymerteilchen und die zahlreichen Stöße untereinander wird die Flüssigkeit gleichmäßig verteilt. Die Oberflächen der Polymerteilchen werden mit Flüssigkeit gleichmäßig benetzt.

Bei dem ersten Mischvorgang treten große dynamische Drücke auf, die durch die hohen Geschwindigkeiten der Polymerteilchen bewirkt werden. Die quasi statischen Drücke sind jedoch vergleichsweise klein.

Durch den zweiten Mischvorgang reifen die Polymerteilchen in der Weise, dass sich die Flüssigkeit im Inneren der Polymerteilchen verteilt.

Durch die geringere Geschwindigkeit der Polymerteilchen beim zweiten Mischvorgang wird eine Verklumpung der Polymerteilchen bzw. eine Agglomeration der Polymerteilchen vermieden. Vorteilhafterweise wird die Geschwindigkeit im zweiten Mischvorgang so klein gewählt, dass der quasi statische Kompressionsdruck gering gehalten wird. In der Folge wird eine Verklumpung verhindert.

In dem erfindungsgemäßen Verfahren liegt zwischen dem ersten Mischvorgang und dem zweiten Mischvorgang ein Reifevorgang, in dem die Polymerteilchen weniger als in dem ersten und zweiten Mischvorgang gegeneinander bewegt werden. Die Polymerteilchen ruhen in dem Reifevorgang. In dem Reifevorgang befinden sich die Polymerteilchen vorzugsweise über einen Zeitraum von 1 Sekunde bis 10 Stunden, vorzugsweise 1,1 Sekunden bis 60 Minuten und besonders bevorzugt 1,5 Sekunden bis 20 Minuten.

In einer anderen Ausgestaltung des erfindungsgemäßen Verfahrens beträgt die mittlere Geschwindigkeit der Polymerteilchen in dem ersten Mischvorgang zwischen 8 und 80 m/sec, insbesondere 10 und 70 m/sec, darüber hinaus bevorzugt zwischen 15 und 60 m/sec.

Durch derartig hohe mittlere Geschwindigkeiten beim ersten Mischvorgang werden bei der typischen Dichte des absorbierenden Polymergranulats bzw. des Gewichtes des einzelnen Polymerteilchens ausreichend große Bewegungsenergien erzielt. Diese großen Bewegungsenergien wirken dem Bestreben der Polymerteilchen zuwider, aneinander haften zu bleiben. Das Bestreben der Polymerteilchen, aneinander haften zu bleiben, ist charakterisiert durch die Haftungsenergie. Die Größe der Haftungsenergie hängt von einer Vielzahl von Parameter ab, u.a. von der Kohäsion der Flüssigkeit, der Adhäsion zwischen Flüssigkeit und Polymerteilchen sowie der Kohäsion zwischen zwei (ggf. teils angequollenen) Polymerteilchen. Die Haftungsenergie ist im wesentlichen dadurch gegeben, wie viel Energie notwendig ist, um zwei aneinander haftende Polymerteilchen von einander zu trennen.

Der zweite Mischvorgang erfolgt mit hinreichend kleinen quasi statischen Drücken. Große statische Drücke haben eine Verklumpung der Polymerteilchen zur Folge. Um hohe statische Drücke zu vermeiden werden entsprechende Mischertypen wie z.B. Schneckenmischer verwendet. Weiterhin kann der zweite Mischvorgang in einer Trommel erfolgen, die gemäß einer Ausführungsform Mischpaddel an der Trommelwand aufweist. Derartige Mischertypen sind in der Herstellung preiswert und im Betrieb sparsam.

Die Höhe der Geschwindigkeiten beim ersten Mischvorgang als auch die Geschwindigkeiten beim zweiten Mischvorgang hängt von der bestimmten Art des absorbierenden Polymers ab. Die Geschwindigkeiten lassen sich aus statischen Überlegungen sowie aus einfachen Messungen bestimmen. Beispielsweise wird die minimale Geschwindigkeit, die für den ersten Mischvorgang erforderlich ist, bestimmt aus der mittleren kinetischen Energie, die notwendig ist um die Haftungsenergie zu überwinden. Die Haftungsenergie ist die Energie, die erforderlich ist, um zwei miteinander verklumpte Polymerteilchen voneinander zu trennen. Sie kann durch eine Messung bestimmt werden. Durch Vorgabe dieser Geschwindigkeiten für die jeweiligen Mischvorgänge wird bewirkt, dass die Polymerteilchen nicht aneinander haften bleiben. Wenn die Polymerteilchen nicht verklumpen bleibt die Oberfläche des Polymer Granulats maximal, wodurch eine schnelle Wasseraufnahme bewirkt wird.

In einer weiteren Ausgestaltung des erfindungsgemäßen Verfahrens beträgt die mittlere Geschwindigkeit der Polymerteilchen im zweiten Mischvorgang unter 3 m/sec, insbesondere unter 0,3 m/sec, vorzugsweise unter 0,03 m/sec. Durch diese niedrigen Geschwindigkeiten wird eine Agglomeration der Polymerteilchen vermieden.

Im erfindungsgemäßen Verfahren zur Herstellung eines absorbierenden Polymers beträgt die Froude-Zahl beim ersten Mischvorgang zwischen 1 und 50, insbesondere zwischen 1,1 und 45, darüber hinaus bevorzugt zwischen 1,5 und 40, besonders bevorzugt zwischen 1,7 und 33 und darüber hinaus noch mehr bevorzugt zwischen 10 und 33. In einer besonderen Ausführungsform des erfindungsgemäßen Verfahrens beträgt die Froude-Zahl beim ersten Mischvorgang mindestens 5, bevorzugt mindestens 10, besonders bevorzugt mindestens 15, darüber hinaus bevorzugt mindestens 20 und darüber hinaus noch mehr bevorzugt mindestens 25, wobei vorzugsweise ein Wert von 60 nicht überschritten wird. Werden zu große Froude-Zahlen bzw. Geschwindigkeiten gewählt, werden die Polymerteilchen nachteilig verändert.

Die Froude-Zahl beim zweiten Mischvorgang beträgt erfindungsgemäß zwischen 0,001 und 1, insbesondere zwischen 0,01 und 0,2, vorzugsweise zwischen 0,08 und 0,03.

Die Froude-Zahl ist eine charakteristische Kennzahl, die bei Rotationsmischern unabhängig von den Abmaßen des Mischers die Mischwirkung des Mischers bestimmt. Die Froude-Zahl $Fr = \dfrac{R \times \omega^2}{g}$ gibt die auf die Erdbeschleunigung normierte Zentrifugalbeschleunigung des zu mischenden Gutes an, wobei R der Radius des Rotationsmischers, $\omega$ die Drehzahl des Rotationsmischers und g die Gravitationskonstante (=9.81 m/sec$^2$) ist. Durch Angabe der Froude-Zahl kann unabhängig von der einzelnen Realisierung des Mischers geräteunabhängig eine Mischwirkung durch Angabe einer normierten Beschleunigung angegeben werden.

Die für die Fluidisierung im ersten Mischvorgang erforderliche Froude-Zahl wird erfahrungsgemäß bei typischen superabsorbierenden Polymeren bei Froude-Zahlen zwischen 1,7 und 33 erreicht. Bei Polymerteilchen, die eine geringere Dichte und damit eine geringere Masse aufweisen, sind unter der Annahme, dass die Haftungsenergie gleich ist, entsprechend größere Froude-Zahlen anzusetzen. Je leichter die Polymerteilchen sind, desto höher müssen ihre Geschwindigkeiten sein, damit eine Haftung bzw. eine Verklumpung durch die Bewegung der Polymerteilchen aufgebrochen wird. Bei schwereren Polymerteilchen sind entsprechend geringere Geschwindigkeiten bzw. kleinere Froude-Zahlen erforderlich.

Der zweite Mischvorgang erfordert geringere Geschwindigkeiten, die kleineren Froude-Zahlen entsprechen. Wichtig beim zweiten Mischvorgang ist, dass nur geringe statische Drücke auftreten, die eine Verklumpung der Polymerteilchen zur Folge hätten.

In einer besonderen Ausgestaltung des erfindungsgemäßen Verfahrens werden im ersten Mischvorgang die Polymerteilchen rückvermischt. Rückvermischung bedeutet, dass ältere Polymerteilchen, die schon eine Weile vermischt worden sind, mit frisch von Flüssigkeit benetzten Polymerteilchen versetzt werden. Dieses geschieht vorzugsweise in einem kontinuierlichen Mischprozess. In einem solchen Mischprozess wird der Strom der in den Mischer eintretenden neuen Polymerteilchen durch einen diesem Strom entgegengesetzen Strom bereits im Mischer vorhandener Polymerteilchen überlagert. Vorteilhafterweise beträgt das Verhältnis des entgegengesetzten Stroms zu dem Strom der neu eintretenden Polymerteilchen, i.e. Rückvermischung, 5 % bis 50 %, vorzugsweise 10 % bis 30 % und besonders bevorzugt 15 % bis 25 %. Die Rückvermischung wird durch eine geeignete Stellung der Mischorgane (Propeller) des Mischers bewirkt. Durch die Rückvermischung wird bewirkt, dass eine besonders gleichmäßige Benetzung der Polymerteilchen mit Flüssigkeit erreicht wird. Durch eine besonders gleichmäßige Homogenisierung der Flüssigkeit im Inneren der Polymerteilchen wird dazu beigetragen, dass die Polymerteilchen sich durch schnelle Flüssigkeitsaufnahme auszeichnen.

In einer speziellen Ausgestaltung des Verfahrens beträgt die mittlere Verweilzeit des ersten Mischvorgangs zwischen 5 und 200 sec, insbesondere zwischen 10 und 100 sec, vorzugsweise zwischen 20 und 60 sec. Unter der mittleren Verweilzeit des ersten Mischvorgangs wird dabei vorzugsweise die Länge des Zeitintervalls verstanden, in dem die Polymerteilchen im Mittel während des ersten Mischvorgangs mit einer solchen Geschwindigkeit mit einer Flüssigkeit vermischt werden, dass die Bewegungsenergie der Polymerteilchen im Mittel größer ist als die Haftungsenergie zwischen den einzelnen Polymerteilchen. Durch die kurze Verweilzeit der Polymerteilchen im ersten Mischvorgang wird ein besonders ökonomischer Betrieb des für den ersten Mischvorgang verwendeten Mischers gewährleistet. Außerdem kann durch diese geringe Dauer die Bildung von durch mechanischen Abrieb gebildeten Feinteilen verhindert werden.

In einer besonderen Ausgestaltung des erfindungsgemäßen Verfahrens beträgt der statische Druckaufbau während des ersten Mischvorgangs weniger als 0,1 bar, insbesondere weniger als 0,05 bar, vorzugsweise weniger als 0,01 bar. Durch diese geringen statischen Drücke wird eine Verklumpung der Polymerteilchen vermieden. Durch Vermeidung einer Verklumpung wird eine große Oberfläche bereit gestellt, die eine schnelle Flüssigkeitsaufnahme erlaubt.

Zur Erhöhung der Absorptionsgeschwindigkeit wird vorteilhafter Weise als Flüssigkeit Wasser oder wässrige Lösung zugegeben. In einer speziellen Ausgestaltung der Erfindung enthält die Flüssigkeit Zusatzstoffe, insbesondere Alkohole. In einer anderen speziellen Ausgestaltung beinhaltet die Flüssigkeit keine Zusatzstoffe. In dieser speziellen Ausgestaltung des erfindungsgemäßen Verfahrens ist die Flüssigkeit insbesondere frei von Additiven wie ein- oder mehrwertigen Metallkationen oder wasserlöslichen organischen Substanzen mit einer Viskosität in einem Bereich zwischen 200 und 300 centistokes, wobei die Flüssigkeit insbesondere frei ist von denjenigen Substanzen, die in der WO-A-98/49221 konkret als Additive bezeichnet werden. Frei von Additiven im Sinne der vorliegenden Erfindungen bedeutet dabei, dass die Flüssigkeit neben der Flüssigkeit als Hauptkomponente diese Additive in Mengen von weniger als 500 ppm , vorzugsweise weniger als 100 ppm, besonders bevorzugt weniger als 10 ppm, darüber hinaus noch mehr bevorzugt weniger als 1 ppm und am meisten bevorzugt weniger als 0,1 ppm beinhaltet.

Die in dem erfindungsgemäßen Verfahren eingesetzten Polymerteilchen weisen vorzugsweise eine mittlere Teilchengrösse gemäß ERT 420.1-99 im Bereich von 10 bis 10000 $\mu$m, besonders bevorzugt im Bereich von 50 bis 5000 $\mu$m und darüber hinaus bevorzugt im Bereich von 100 bis 1000 $\mu$m auf.

Es ist weiterhin bevorzugt, dass die in dem erfindungsgemäßen Verfahren eingesetzten Polymerteilchen auf

($\alpha$1) 0,1 bis 99,999 Gew.-%, bevorzugt 20 bis 98,99 Gew.-% und besonders bevorzugt 30 bis 98,95 Gew.-% polymerisierten, ethylenisch ungesättigten, säuregruppenhaltigen Monomeren oder deren Salzen oder polymerisierten, ethylenisch ungesättigten, einen protonierten oder quarternierten Stickstoff beinhaltenden Monomeren, oder deren Mischungen, wobei mindestens ethylenisch ungesättigte, säuregruppenhaltige Monomere, vorzugsweise Acrylsäure beinhaltende Mischungen besonders bevorzugt sind,

($\alpha$2) 0 bis 70 Gew.-%, bevorzugt 1 bis 60 Gew.-% und besonders bevorzugt 1 bis 40 Gew.-% polymerisierten, ethylenisch ungesättigten, mit ($\alpha$1) copolymerisierbaren Monomeren,

($\alpha$3) 0,001 bis 10 Gew.-%, bevorzugt 0,01 bis 7 Gew.-% und besonders bevorzugt 0,05 bis 5 Gew.-% eines oder mehrerer Vernetzer,

($\alpha$4) 0 bis 30 Gew.-%, bevorzugt 1 bis 20 Gew.-% und besonders bevorzugt 5 bis 10 Gew.-% wasserlöslichen Polymeren, sowie

($\alpha$5) 0 bis 20 Gew.-%, bevorzugt 0,01 bis 7 Gew.-% und besonders bevorzugt 0,05 bis 5 Gew.-% eines oder mehrerer Hilfsstoffe basieren, wobei die Summe der Gewichtsmengen ($\alpha$1) bis ($\alpha$5) 100 Gew.-% beträgt.

Die monoethylenisch ungesättigten, säuregruppenhaltigen Monomere ($\alpha$1) können teilweise oder vollständig, bevorzugt teilweise neutralisiert sein. Vorzugsweise sind die monoethylenisch ungesättigten, säuregruppenhaltigen Monomere zu mindestens 25 Mol%, besonders bevorzugt zu mindestens 50 Mol% und darüber hinaus bevorzugt zu 50-90 Mol% neutralisiert. Die Neutralisation der Monomere ($\alpha$1) kann vor oder auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als *"Mixed Bed Ion-Exchange Absorbent Polymers"* (MBIEA-Polymere) bezeichnet und sind unter anderem in der WO 99/34843 offenbart. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melanin und dergleichen, sowie deren sekundäre oder tertiäre Aminderivate.

Bevorzugte monoethylenisch ungesättigte, säuregruppenhaltige Monomere ($\alpha$1) sind Acrylsäure, Methacrylsäure, Ethacrylsäure, $\alpha$-Chloracrylsäure, $\alpha$-Cyanoacrylsäure, $\beta$-Methylacrylsäure (Crotonsäure), $\alpha$-Phenylacrylsäure, $\beta$-Acryloxypropionsäure, Sorbinsäure, $\alpha$-Chlorsorbinsäure, 2'-Methylisocrotonsäure, Zimtsäure, p-Chlorzimtsäure, $\beta$-Stearylsäure, Itaconsäure, Citraconsäure, Mesaconsäure, Glutaconsäure, Aconitsäure, Maleinsäure, Fumarsäure, Tricarboxyethylen und Maleinsäureanhydrid, wobei Acrylsäure sowie Methacrylsäure besonders und Acrylsäure darüber hinaus bevorzugt sind.

Neben diesen carboxylatgruppenhaltigen Monomeren sind als monoethylenisch ungesättigte, säuregruppenhaltige Monomere ($\alpha$1) des Weiteren ethylenisch ungesättigte Sulfonsäuremonomere oder ethylenisch ungesättigte Phosphonsäuremonomere bevorzugt.

Ethylenisch ungesättigte Sulfonsäuremonomere sind Allylsulfonsäure oder aliphatische oder aromatische Vinylsulfonsäuren oder acrylische oder methacrylische Sulfonsäuren bevorzugt. Als aliphatische oder aromatische Vinylsulfonsäuren sind Vinylsulfonsäure, 4-Vinylbenzylsulfonsäure, Vinyltoluolsulfonsäure und Stryrolsulfonsäure bevorzugt. Als Acryl- bzw. Methacrylsulfonsäuren sind Sulfoethyl(meth)acrylat, Sulfopropyl(meth)acrylat und, 2-Hydroxy-3-methacryloxypropylsulfonsäure. Als (Meth)Acrylamidoalkylsulfonsäure ist 2-Acrylamido-2-methylpropansulfonsäure bevorzugt.

Ferner sind ethylenisch ungesättigte Phosphonsäuremonomere, wie Vinylphosphonsäure, Allylphosphonsäure, Vinylbenzylphosphonsäure, (Meth)acrylamidoalkylphosphonsäuren, Acrylamidoalkyldiphosphonsäuren, phosponomethylierte Vinylamine und (Meth)acrylphosphonsäurederivate bevorzugt.

Als ethylenisch ungesättigte, einen protonierten Stickstoff enthaltende Monomere ($\alpha$1) sind Dialkylaminoalkyl(meth)acrylate in protonierter Form, beispielsweise Dimethylaminoethyl(meth)acrylat-Hydrochlorid oder Dimethylaminoethyl(meth)acrylat-Hydrosulfat, sowie Dialkylaminoalkyl-(meth)acrylamide in protonierter Form, beispielsweise Dimethylaminoethyl(meth)acrylamid-Hydrochlorid, Diemethylaminopropyl(meth)acrylamid-Hydrochlorid, Dimethylaminopropyl(meth)acrylamid-Hydrosulfat oder Dimethylaminoethyl(meth)acrylamid-Hydrosulfat bevorzugt.

Als ethylenisch ungesättigte, einen quarternierten Stickstoff enthaltende Monomere (α1) sind Dialkylammoniumalkyl(meth)acrylate in quarternisierter Form, beispielsweise Trimethylammoniumethyl(meth)acrylat-Methosulfat oder Dimethylethylammoniumethyl(meth)acrylat-Ethosulfat sowie (Meth)acrylamidoalkyldialkylamine in quarternisierter Form, beispielsweise (Meth)acrylamidopropyltrimethylammoniumchlorid, Trimethylammoniumethyl(meth)acrylat-Chlorid oder (Meth)acrylamidopropyltrimethylammoniumsulfat bevorzugt.

Es ist erfindungsgemäß bevorzugt, dass das Polymer zu mindestens 50 Gew.-%, vorzugsweise zu mindestens 70 Gew.-% und darüber hinaus bevorzugt zu mindestens 90 Gew.-% auf carboxylatgruppenhaltigen Monomeren besteht. Es ist erfindungsgemäß besonders bevorzugt, dass das Polymer zu mindestens 50 Gew.-%, vorzugsweise zu mindestens 70 Gew.-% aus Acrylsäure besteht, die vorzugsweise zu mindestens 20 Mol-%, besonders bevorzugt zu mindestens 50 Mol-% neutralisiert ist.

Als monoethylenisch ungesättigte, mit (α1) copolymerisierbare Monomere (α2) sind Acrylamide und Methacrylamide bevorzugt.

Mögliche (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethyl-amino(meth)acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Des Weiteren sind als monoethylenisch ungesättigte, mit (α1) copolymerisierbaren Monomere (α2) in Wasser dispergierbare Monomere bevorzugt. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)acrylat, sowie Vinylacetat, Styrol und Isobutylen bevorzugt.

Erfindungsgemäß bevorzugte Vernetzer (α3) sind Verbindungen, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren (α1) oder (α2) in einer Kondensationsreaktion (=Kondensationsvernetzer), in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren (α1) oder (α2) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV). Dabei wird durch die Verbindungen der Vernetzerklasse I eine Vernetzung der Polymere durch die radikalische Polymerisation der ethylenisch ungesättigten Gruppen des Vernetzermoleküls mit den monoethylenisch ungesättigten Monomeren (α1) oder (α2) erreicht, während bei den Verbindungen der Vernetzerklasse II und den polyvalenten Metallkationen der Vernetzerklasse IV eine Vernetzung der Polymere durch Kondensationsreaktion der funktionellen Gruppen (Vernetzerklasse II) bzw. durch elektrostatische Wechselwirkung des polyvalenten Metallkations (Vernetzerklasse IV) mit den funktionellen Gruppen der Monomere (α1) oder (α2) erreicht wird. Bei den Verbindungen der Vernetzerklasse III erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren (α1) oder (α2).

Bevorzugte Verbindungen der Vernetzerklasse I sind Poly(meth)acrylsäureester, die beispielsweise durch die Umsetzung eines Polyols, wie beispielsweise Ethylenglykol, Propylenglykol, Trimethylolpropan, 1,6-Hexandiol, Glycerin, Pentaerythrit, Polyethylenglykol oder Polypropylenglykol, eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Als Verbindungen der Vernetzerklasse I sind des Weiteren Polyvinylverbindungen, Poly(meth)allylverbindungen, (Meth)acrylsäureester einer Monovinylverbindung oder (Meth)acrylsäureester einer Mono(meth)allylverbindung, vorzugsweise der Mono(meth)allylverbindungen eines Polyols oder eines Aminoalkohols, bevorzugt. In diesem Zusammenhang wird auf DE 195 43 366 und DE 195 43 368 verwiesen.

Als Verbindungen der Vernetzerklasse I seien als Beispiel genannt Alkenyldi(meth)acrylate, beispielsweise Ethylenglykoldi(meth)acrylat, 1,3-Propylenglykoldi(meth)acrylat, 1,4-Butylenglykoldi(meth)acrylat, 1,3-Butylenglykoldi(meth)acrylat, 1,6-Hexandioldi(meth)acrylat, 1,10-Decandioldi(meth)acrylat, 1,12-Dodecandioldi(meth)acrylat, 1,18-Octadecandioldi(meth)acrylat, Cyclopentandioldi(meth)acrylat, Neopentylglykoldi(meth)acrylat, Methylendi(meth)acrylat oder Pentaerythritdi(meth)acrylat, Alkenyldi(meth)acrylamide, beispielsweise N-Methyldi(meth)acrylamid, N,N'-3-Methylbutylidenbis(meth)acrylamid, N,N'-(1,2-Di-hydroxyethylen)bis(meth)acrylamid, N,N'-Hexamethylenbis(meth)acrylamid oder N,N'-Methylenbis(meth)acrylamid, Polyalko-xydi(meth)acrylate, beispielsweise Diethylenglykoldi(meth)acrylat, Triethylenglykoldi(meth)acrylat, Tetraethylenglykoldi(meth)acrylat, Dipropylenglykoldi(meth)acrylat, Tripropylenglykoldi(meth)acrylat oder Tetrapropylenglykoldi(meth)acrylat, Bisphenol-A-di(meth)acrylat, ethoxyliertes Bisphenol-A-di(meth)acrylat, Benzylidindi(meth)acrylat, 1,3-Di(meth)acryloyloxy-propanol-2, Hydrochinondi(meth)acrylat, Di(meth)acrylatester des vorzugsweise mit 1 bis 30 Mol Alkylenoxid pro Hydroxylgruppe oxyalkylierten, vorzugsweise ethoxylierten Trimethylolpropans, Thioethylenglykoldi(meth)acrylat, Thiopropylenglykoldi(meth)acrylat, Thiopolyethylenglykoldi(meth)acrylat, Thiopolypropylenglykoldi(meth)acrylat, Divinylether, beispielsweise 1,4-Butandioldivinylether, Divinylester, beispielsweise Divinyladipat, Alkandiene, beispielsweise Butadien oder 1,6-Hexadien, Divinylbenzol, Di(meth)allylverbindungen, beispielsweise Di(meth)allylphthalat oder Di(meth)allylsuccinat, Homo- und Copolymere von Di(meth)allyldimethylammoniumchlorid und Homo- und Copolymere von Diethyl(meth)allylaminomethyl(meth)acrylatammoniumchlorid, Vinyl-(meth)acryl-Verbindungen, beispielsweise Vinyl(meth)acrylat, (Meth)allyl-(meth)acryl-Verbindungen, beispielsweise (Meth)allyl(meth)acrylat, mit 1 bis 30 Mol Ethylenoxid pro Hydroxylgruppe ethoxyliertes (Meth)allyl(meth)acrylat, Di(meth)allylester von Polycarbonsäuren, beispielsweise Di(meth)allylmaleat, Di(meth)allylfumarat, Di(meth)allylsuccinat oder Di(meth)allylterephthalat, Verbindungen mit 3 oder mehr ethylenisch ungesättigten, radikalisch polymerisierbaren Gruppen wie beispielsweise Glycerintri(meth)acrylat, (Meth)acrylatester des mit vorzugsweise 1 bis 30 Mol Ethylenoxid pro Hydroxylgruppe oxyethylierten Glycerins, Trimethylolpropantri(meth)acrylat, Tri(meth)acrylatester des vorzugsweise mit 1 bis 30 Mol Alkylenoxid pro Hydroxylgruppe oxyalkylierten, vorzugsweise ethoxylierten Trimethylolpropans, Trimethacrylamid, (Meth)allylidendi(meth)acrylat, 3-Allyloxy-1,2-propandioldi(meth)acrylat, Tri(meth)allylcyanurat, Tri(meth)allylisocyanurat, Pentaerythrittetra(meth)acrylat, Pentaerythrittri(meth)acrylat, (Meth)acrylsäureester des mit vorzugsweise 1 bis 30 Mol Ethylenoxid pro Hydroxylgruppe oxyethylierten Pentaerythrits, Tris(2-hydroxyethyl)isocyanurattri(meth)acrylat, Trivinyltrimellitat, Tri(meth)allylamin, Di(meth)allylalkylamine, beispielsweise Di(meth)allylmethylamin, Tri(meth)allylphosphat Tetra(meth)allylethylendiamin, Poly(meth)allylester, Tetra(meth)allyloxiethan oder Tetra(meth)allylammoniumhalide.

Als Verbindung der Vernetzerklasse II sind Verbindungen bevorzugt, die mindestens zwei funktionelle Gruppen aufweisen, die in einer Kondensationsreaktion (=Kondensationsvernetzer), in einer Additionsreaktion oder in einer Ringöffnungsreaktion mit den funktionellen Gruppen der Monomere ($\alpha$1) oder ($\alpha$2), bevorzugt mit Säuregruppen, der Monomeren ($\alpha$1), reagieren können. Bei diesen funktionellen Gruppen der Verbindungen der Vernetzerklasse II handelt es sich vorzugsweise um Alkohol-, Amin-, Aldehyd-, Glycidyl-, Isocyanat-, Carbonat- oder Epichlorfunktionen.

Als Verbindung der Vernetzerklasse II seien als Beispiele genannt Polyole, beispielsweise Ethylenglykol, Polethylenglykole wie Diethylenglykol, Triethylenglykol und Tetraethylenglykol, Propylenglykol, Polypropylenglykole wie Dipropylenglykol, Tripropylenglykol oder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin, Diethanolamin, Triethanolamin oder Propanolamin, Polyaminverbindungen, beispielsweise Ethylendiamin, Diethylentriamin, Triethylentetraamin, Tetraethylenpentaamin oder Pentaethylenhexaamin, Polyglycidylether-Verbindungen wie Ethylenglykoldiglycidylether, Polyethylenglykoldiglycidylether, Glycerindiglycidylether, Glycerinpolyglycidylether, Pentaeritritpolyglycidylether, Propylenglykoldiglycidylether Polypropylenglykoldiglycidylether, Neopentylglykoldiglycidylether, Hexandiolglycidylether, Trimethylolpropanpolyglycidylether, Sorbitolpolyglycidylether, Phthalsäurediglycidylester, Adipinsäurediglycidylester, 1,4-Phenylen-bis(2-oxazolin), Glycidol, Polyisocyanate, vorzugsweise Diisocyanate wie 2,4-Toluoldiisocyanat und Hexamethylendiisocyanat, Polyaziridin-Verbindungen wie 2,2-Bishydroxymethylbutanol-tris[3-(1-aziridinyl)propionat], 1,6-Hexamethylendiethylenharnstoff und Diphenylmethan-bis-4,4'-N,N'-diethylenharnstoff, Halogenepoxide beispielsweise Epichlor- und Epibromhydrin und $\alpha$-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, polyquartäre Amine wie Kondensationsprodukte von Dimethylaminen und Epichlorhydrin. Als Verbindungen der Vernetzerklasse II sind des weiteren Polyoxazoline wie 1,2-Ethylenbisoxazolin, Vernetzer mit Silangruppen wie $\gamma$-Glycidoxypropyltrimethoxysilan und $\gamma$-Aminopropyltrimethoxysilan, Oxazolidinone wie 2-Oxazolidinon, Bis- und Poly-2-oxazolidinone und Diglykolsilikate bevorzugt.

Als Verbindungen der Klasse III sind hydroxyl- oder aminogruppenhaltige Ester der (Meth)acrylsäure, wie beispielsweise 2-Hydroxyethyl(meth)acrylat, sowie hydroxyl- oder aminogruppenhaltige (Meth)acrylamide, oder Mono(meth)allylverbindungen von Diolen bevorzugt.

Die polyvalenten Metallkationen der Vernetzerklasse IV leiten sich vorzugsweise von ein- oder vorzugsweise mehrwertigen Kationen ab. Besonders bevorzugte zweiwertige Kationen leiten sich von Zink, Beryllium, Erdalkalimetallen, wie Magnesium, Calcium, Strontium ab, wobei Magnesium bevorzugt wird. Weiter erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z. B. $AlCl_3 \times 6H_2O$, $NaAl(SO_4)_2 \times 12\ H_2O$, $KAl(SO_4)_2 \times 12\ H_2O$ oder $Al_2(SO_4)_3 \times 14\text{-}18\ H_2O$ eingesetzt.

Besonders bevorzugt werden $Al_2(SO_4)_3$ und seine Hydrate als Vernetzer der Vernetzungsklasse IV verwendet.

Bevorzugte Polymerteilchen sind Polymerteilchen, die durch Vernetzer der folgenden Vernetzerklassen bzw. durch Vernetzer der folgenden Kombinationen von Vernetzerklassen vernetzt sind: I, II, III, IV, I II, I III, I IV, I II III, I II IV, I III IV, II III IV, II IV oder III IV. Die vorstehenden Kombinationen von Vernetzerklassen stellen jeweils eine bevorzugte Ausführungsform von Vernetzern eines Polymerteilchens dar.

Weitere bevorzugte Ausführungsformen der Polymerteilchen sind Polymerteilchen, die durch einen beliebigen der vorstehend genannten Vernetzer der Vernetzerklassen I vernetzt sind. Unter diesen sind wasserlösliche Vernetzer bevorzugt. In diesem Zusammenhang sind N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Aus den vorgenannten Monomeren und Vernetzern lassen sich die wasserabsorbierenden Polymere durch verschiedene Polymerisationsweisen herstellen. Beispielsweise sind in diesem Zusammenhang Massepolymerisation, die vorzugsweise in Knetreaktoren wie Extrudern oder durch Bandpolymerisation erfolgt, Lösungspolymerisation, Spraypolymerisation, inverse Emulsionspolymerisation und inverse Suspensionspolymerisation zu nennen. Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818.

Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der obengenannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere Peroxide, Hydroperoxide, Wasserstoffperoxid, Persulfate, Azoverbindungen sowie die sogenannten Redoxkatalysatoren. Bevorzugt ist der Einsatz wasserlöslicher Katalysatoren. In manchen Fällen ist es vorteilhaft, Mischungen verschiedener Polymerisationsinitiatoren zu verwenden. Unter diesen Mischungen sind die aus Wasserstoffperoxid und Natrium- oder Kaliumperoxodisulfat bevorzugt, die in jedem denkbaren Mengenverhältnis eingesetzt werden können. Geeignete organische Peroxide sind vorzugsweise Acetylacetonperoxid, Methylethylketonperoxid, t-Butylhydroperoxid, Cumolhydroperoxid, t-Amylperpivalat, t-Butylperpivalat, t-Butylperneohexonat, t-Butylisobutyrat, t-Butylper-2-ethylhexenoat, t-Butylperisononanoat, t-Butylpermaleat, t-Butylperbenzoat, tButyl-3,5,5-tri-methylhexanoat und Amylperneodekanoat. Weiterhin sind als Polymerisationsinitiatoren bevorzugt: Azo-Verbindungen, wie 2,2'-Azobis-(2amidinopropan)dihydrochlorid, Azo-bis-amidinopropan-dihydrochlorid, 2,2'-Azobis-(N,N-dimethylen)isobutyramidin-dihydrochlorid, 2-(Carbamoylazo)isobutyronitril und 4,4'-Azobis-(4-cyanovaleriansäure). Die genannten Verbindungen werden in üblichen Mengen eingesetzt, vorzugsweise in einem Bereich von 0,01 bis 5, bevorzugt von 0,1 bis 2 Mol-%, jeweils bezogen auf die Menge der zu polymerisierenden Monomere.

Die Redoxkatalysatoren enthalten als oxidische Komponente mindestens eine der oben angegebenen Perverbindungen und als reduzierende Komponente vorzugsweise Ascorbinsäure, Glukose, Sorbose, Mannose, Ammonium- oder Alkalimetall-hydrogensulfit, -sulfat, -thiosulfat, -hyposulfit oder -sulfid, Metallsalze, wie Eisen-II-ionen oder Silberionen oder Natriumhydroxymethylsulfoxylat. Vorzugsweise wird als reduzierende Komponente des Redoxkatalysators Ascorbinsäure oder Natriumpyrosulfit verwendet. Bezogen auf die bei der Polymerisation eingesetzte Menge an Monomeren wird $1 \times 10^{-5}$ bis 1 Mol-% der reduzierenden Komponente des Redoxkatalysators und $1 \times 10^{-5}$ bis 5 Mol-% der oxidierenden Komponente des Redoxkatalysators eingesetzt. Anstelle der oxidierenden Komponente des Redoxkatalysators, oder in Ergänzung zu diesem, können ein oder mehrere, vorzugsweise wasserlösliche, Azoverbindungen verwendet werden.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte α -Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1naphthyl-2'-(N,N-dimethylamino)ethylsulfon, N-(4-Sulfonylazidophe-nyl)maleinimid, N-Acetyl-4-sulfonylazidoanilin, 4-Sulfonylazidoanilin, 4-Azido-anilin, 4-Azidophenacylbromid, p-Azidobenzoesäure, 2,6-Bis(p-azidobenzyli-den)cyclohexanon und 2,6-Bis-(p-azidobenzyliden)-4-methylcyclohexanon. Die Photoinitiatoren werden, falls sie eingesetzt werden, üblicherweise in Mengen von 0,01 bis 5 Gew.-%, bezogen auf die zu polymerisierenden Monomeren angewendet.

Bevorzugt wird erfindungsgemäß ein Redoxsystem bestehend aus Wasserstoffperoxid, Natriumperoxodisulfat und Ascorbinsäure eingesetzt. Allgemein sind erfindungsgemäß Azoverbindungen als Initiatoren bevorzugt, wobei Azo-bis-amidinopropan-dihydrochlorid besonders bevorzugt ist. In der Regel wird die Polymerisation mit den Initiatoren in einem Temperaturbereich von 30 bis 90°C initiiert.

Nach der Herstellung der wasserabsorbierenden Polymere werden diese getrocknet und unter Bildung der vorstehend beschriebenen Polymerteilchen zerkleinert.

Als wasserlösliche Polymere (α4) können in den Polymerteilchen wasserlösliche Polymerisate, wie teil- oder vollverseifter Polyvinylalkohol, Polyvinylpyrrolidon, Stärke oder Stärkederivate, Polyglykole oder Polyacrylsäure enthalten, vorzugsweise einpolymerisiert sein. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische Polymere wie Polyvinylalkohol, können auch als Pfropfgrundlage für die zu polymerisierenden Monomeren dienen.

Als Hilfsstoffe (α5) sind in den Polymerteilchen vorzugsweise Stellmittel, Geruchsbinder, oberflächenaktive Mittel oder Antioxidatien enthalten. Diese Hilfsstoffe (α5) werden vorzugsweise vor der Polymerisation der Monomerenlösung zugesetzt oder aber nach Herstellung der Polymerteilchen mit diesen vermischt, wobei für das Vermischen die dem Fachmann bekannten Mischaggregate verwendet werden können, z. B. der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen die Polymerteilchen und die Hilfsstoffe (α5) mittels rotierender Messer in schneller Frequenz gemischt wird (Schugi-Mischer).

In einer anderen Ausführungsformen des erfindungsgemäßen Verfahrens wird der Außenbereich der absorbierenden Polymerteilchen mit einer Verbindung enthaltend ein $Al^{3+}$-Ion in Kontakt gebracht. Dabei ist es bevorzugt, dass die Verbindung enthaltend $Al^{3+}$-Ionen in einer Menge in einem Bereich von 0,01 bis 30 Gew.-%, besonders bevorzugt in einer Menge in einem Bereich von 0,1 bis 20 Gew.-% und darüber hinaus bevorzugt in einer Menge in einem Bereich von 0,3 bis 5 Gew.-%, jeweils bezogen auf das Gewicht der absorbierenden Polymerteilchen, mit den Polymerteilchen in Kontakt gebracht werden.

Vorzugsweise erfolgt das in Kontakt bringen der absorbierenden Polymerteilchen mit der $Al^{3+}$-Ionen enthaltende Verbindung, in dem diese Verbindung in Form eines Fluids beinhaltend die $Al^{3+}$-Ionen enthaltende Verbindung sowie ein Lösungsmittel, vorzugsweise Wasser, mit Wasser mischbare organische Lösemittel wie etwa Methanol oder Ethanol oder Mischungen aus mindestens zwei davon, mit den absorbierenden Polymerteilchen in Kontakt gebracht wird. Die $Al^{3+}$-Ionen enthaltende Verbindung ist dabei, ohne Berücksichtigung von Kristallwasser, vorzugsweise in einer Menge in einem Bereich von 0,1 bis 50 Gew.-%, bevorzugt in einer Menge im Bereich von 1 bis 30 Gew.-%, jeweils bezogen auf das Gesamtgewicht des Fluids, in dem Fluid enthalten. Weiterhin bevorzugt ist, dass das Fluid in einer Menge in einem Bereich von 0,01 bis 15 Gew.-%, bevorzugt in einer Menge in einem Bereich von 0,05 bis 6 Gew.-%, jeweils bezogen auf das Gewicht des absorbierenden Polymerteilchens mit den absorbierenden Polymerteilchen in Kontakt gebracht wird.

Bevorzugte $Al^{3+}$-Ionen enthaltende Verbindungen sind $AlCl_3$ x 6 $H_2O$, $NaAl(SO_4)_2$ x 12 $H_2O$, $KAl(SO_4)_2$ x 12$H_2O$ oder $Al_2(SO_4)_3$ X 14-18$H_2O$.

In einer weiteren Ausführungsform des erfindungsgemäßen Verfahrens enthält die Flüssigkeit, mit der im ersten Mischvorgang die Polymerteilchen vermischt und die im zweiten Mischvorgang im Inneren der Polymerteilchen homogenisiert wird, eine $Al^{3+}$-Ionen enthaltende Verbindung. Bevorzugte $Al^{3+}$-Ionen enthaltende Verbindungen sind $AlCl_3 \times 6H_2O$, $NaAl(SO_4)_2 \times 12$ $H_2O$, $KAl(SO_4)_2 \times 12$ $H_2O$ oder $Al_2(SO_4)_3 \times 14$-18 $H_2O$.

Die in dem erfindungsgemäßen Verfahren eingesetzten Polymerteilchen weisen einen Innenbereich, einen den Innenbereich umgebenden Aussenbereich sowie einen den Aussenbereich umgebenden Oberflächenbereich auf, wobei der Aussenbereich einen höheren Vernetzungsgrad als der Innenbreich aufweist, so dass sich vorzugsweise eine Kern-Schale-Struktur ausbildet. Die erhöhte Vernetzung im Oberflächenbereich der eingesetzten Polymerteilchen wird dabei vorzugsweise durch Nachvernetzung oberflächennaher, reaktiver Gruppen erreicht. Diese Nachvernetzung kann thermisch, photochemisch oder chemisch erfolgen. Als Nachvernetzer für die chemische Nachvernetzung sind dabei die Verbindungen bevorzugt, die als Vernetzer (α3) der Vernetzerklassen II und IV genannt wurden. Besonders bevorzugt als Nachvernetzer ist Ethylencarbonat.

Vorzugsweise wird der Nachvernetzer in einer Menge in einem Bereich von 0,01 bis 30 Gew.-%, besonders bevorzugt in einer Menge in einem Bereich von 0,1 bis 20 Gew.-% und darüber hinaus bevorzugt in einer Menge in einem Bereich von 0,3 bis 5 Gew.-%, jeweils bezogen auf das Gewicht der in dem erfindungsgemäßen Verfahren eingesetzten Polymerteilchen, zur Nachvernetzung eingesetzt.

Vorzugsweise erfolgt die Nachvernetzung dadurch, dass ein Fluid $F_1$ umfassend ein Lösemittel, vorzugsweise Wasser, mit Wasser mischbare organische Lösemittel wie etwa Methanol oder Ethanol oder Mischungen aus mindestens zwei davon, sowie den Nachvernetzer mit dem Aussenbereich der Polymerteilchen bei einer Temperatur in einem Bereich von 30 bis 300°C, besonders bevorzugt in einem Bereich von 100 bis 200°C in Kontakt gebracht wird. Das in Kontakt bringen erfolgt dabei vorzugsweise durch Aufsprühen des Fluids auf die Polymerteilchen und anschließendes Mischen der mit dem Fluid $F_1$ in Kontakt gebrachten Polymerteilchen. Dabei ist der Nachvernetzer in dem Fluid $F_1$ vorzugsweise in einer Menge in einem Bereich von 0,01 bis 20 Gew.-%, besonders bevorzugt in einer Menge in einem Bereich von 0,1 bis 10 Gew.-%, bezogen auf das Gesamtgewicht des Fluids $F_1$, enthalten. Es ist weiterhin bevorzugt, dass das Fluid $F_1$ in einer Menge in einem Bereich von 0,01 bis 50 Gew.-%, besonders bevorzugt in einer Menge in einem Bereich von 0,1 bis 30 Gew.-%, jeweils bezogen auf das Gewicht der Polymerteilchen, mit den Polymerteilchen in Kontakt gebracht wird.

Sofern die absorbierenden Polymerteilchen mit einer Al$^{3+}$-Ionen enthaltende Verbindung in Kontakt gebracht werden, ist es weiterhin bevorzugt, dass die Polymerteilchen im Falle einer Nachvernetzung vor Durchführung der Nachvernetzung mit der Al$^{3+}$-Ionen enthaltende Verbindung in Kontakt gebracht werden. Dabei können die absorbierenden Polymerteilchen zunächst mit einem Fluid enthaltend die Al$^{3+}$-Ionen enthaltende Verbindung und anschließend mit einem Fluid enthaltend den Nachvernetzer in Kontakt gebracht werden. Denkbar ist auch, die Al$^{3+}$-Ionen enthaltende Verbindung und den Nachvernetzer in einem gemeinsamen Fluid mit den absorbierenden Polymerteilchen in Kontakt zu bringen und anschließend durch Temperaturerhöhung die Nachvernetzung zu bewirken. Entscheidend ist lediglich, dass das in Kontakt bringen der absorbierenden Polymerteilchen mit der Al$^{3+}$-Ionen enthaltende Verbindung vor der Durchführung der Nachvernetzungsreaktion erfolgt.

Es ist weiterhin bevorzugt, dass die in dem erfindungsgemäßen Verfahren eingesetzten Polymerteilchen mindestens eine der folgenden Eigenschaften aufweisen:

(A) die maximale Aufnahme von 0,9 Gew.-%er NaCl-Lösung gemäß ERT 440.1-99 liegt in einem Bereich von mindestens 10 bis 1000, bevorzugt von 15 bis 500 und besonders bevorzugt von 20 bis 300 g/g,

(B) der mit 0,9 Gew.-%er wässriger NaCl-Lösung extrahierbare Anteil gemäß ERT 470.1-99 beträgt weniger als 30, bevorzugt weniger als 20 und besonders bevorzugt weniger als 10 Gew.-%; bezogen auf das Polymerteilchen,

(C) die Schüttdichte gemäß ERT 460.1-99 liegt im Bereich von 300 bis 1000, bevorzugt 310 bis 800 und besonders bevorzugt 320 bis 700 g/l,

(D) der pH-Wert gemäß ERT 400.1-99 von 1 g des Polymerteilchens in 1 l Wasser liegt im Bereich von 4 bis 10, bevorzugt von 5 bis 9 und besonders bevorzugt von 5,5 bis 7,5,

(E) der CRC-Wert nach ERT 441.1-99 liegt im Bereich von 10 bis 100, bevorzugt 15 bis 80 und besonders bevorzugt 20 bis 60 g/g,

(F) der AAP-Wert gemäß ERT 442.1-99 bei einem Druck von 0,7 psi (50 g/cm$^2$) liegt im Bereich von 10 bis 60, bevorzugt 15 bis 50 und besonders bevorzugt 20 bis 40 g/g,

(G) der AAP-Wert gemäß ERT 442.1-99 bei einem Druck von 0,3 psi (20 g/cm$^2$) liegt im Bereich von 10 bis 100, bevorzugt 15 bis 60 und besonders bevorzugt 20 bis 50 g/g.

Die sich aus den vorstehenden Eigenschaften ergebenden Eigenschaftskombinationen von zwei oder mehr dieser Eigenschaften stellen jeweils bevorzugte Ausführungsformen des erfindungsgemässen Verfahrens dar. Weiterhin als erfindungsgemässe Ausführungsformen besonders bevorzugt sind Verfahren, in denen die in dem erfindungsgemäßen Verfahren eingesetzten Polymerteilchen die nachfolgend als Buchstaben oder Buchstabenkombinationen dargestellten Eigenschaften oder Eigenschaftskombinationen zeigen: A, B, C, D, E, F, G, AB, AC, AD, AE, AF, AG, EF, EG, FG, ABC, ABD, ABE, ABF, ABG, ACD, ACE, ACF, ACG, ADE, ADF, ADG, AEF, AEG, CEF, CEG, EFG, ABCD, ABCE, ABCF, ABCG, ABDE, ABDF, ABDG, ACDE, ACDF, ACDG, ACEF, ACEG, ADEF, ADEG, CEFG, ACDEF, ACDEG, ABDEF, ABDEG, ABCEF, ABCEG, ACBDF, ACBDG, ABCDE, ABCDEF, ABCDEG oder ABCDEFG, wobei die Kombinationen CEFG besonders bevorzugt ist und die Kombination EF darüber hinaus bevorzugt ist.

Weitere vorteilhafte Ausgestaltungen und Besonderheiten, die einzeln oder in Kombination auftreten können, werden anhand der folgenden Zeichnung erläutert. Die Zeichnung soll jedoch die Erfindung nicht einschränken, sondern soll nur exemplarisch die Erfindung veranschaulichen. Es zeigen schematisch:

Fig. 1        die beiden erfindungsgemäßen Verfahrensschritte zur Herstellung eines absorbierenden Polymers; und

Fig. 2        einen Stoß zwischen einem Polymerteilchen mit zwei verklebten Polymerteilchen während des ersten Mischvorgangs.

Fig. 3        Prüfeinrichtung zur Bestimmung der Aufnahmezeit (Acquisition Time) im Querschnitt.

Fig. 4        Detailansicht der Prüfeinrichtung zur Bestimmung der Aufnahmezeit (Acquisition Time).

Fig. 5        Darstellung der genauen Abmessungen einzelner Elemente der Prüfeinrichtung zur Bestimmung der Aufnahmezeit (Acquisition Time).

Fig. 6 und 7      Versuchsaufbau zur Bestimmung des Rewet-Wertes.

Fig. 1 zeigt, dass im ersten Mischvorgang ein Polymerteilchen (1) mit Flüssigkeit (2) vermischt wird. Dabei wird die Oberfläche des Polymerteilchens (1) mit Flüssigkeit (2) benetzt und ein mit Flüssigkeit (2) benetztes Polymerteilchen (3) gebildet. In dem zweiten Mischvorgang dringt die Flüssigkeit (2) des benetzten Polymerteilchens (3) in das Innere des Polymerteilchens (1) ein. Damit wird die Flüssigkeit (2) homogen im Polymerteilchen (1) verteilt. Das Polymerteilchen (1) quillt etwas auf. Die Homogenisierung der Flüssigkeit (2) im Polymerteilchen (1) bewirkt eine schnellere Aufnahme von Wasser.

Fig. 2 zeigt als Beispiel einen Stoß zwischen einem Polymerteilchen (1) mit zwei verklebten Polymerteilchen (1', 1"). Die beiden verklebten Polymerteilchen (1', 1") werden durch verschiedenste Faktoren wie z.B. Kohäsion bzw. Adhäsion der Flüssigkeit (2) und der Polymerteilchen (1', 1") aneinander gehalten. Eine Haftschicht (5) zwischen den Oberflächen der beiden Polymerteilchen (1', 1") begründet eine weitere Bindungswirkung zwischen den beiden Polymerteilchen (1', 1"). Durch die hohe Geschwindigkeit des anfliegenden Polymerteilchens (1) wird die Haftung zwischen den beiden verklebten Polymerteilchen (1') und (1") überwunden. Insbesondere wird die Haftschicht (5) aufgebrochen. Das Ergebnis des Stoßes ist eine Separation des einen verklebten Polymerteilchens (1') von dem anderen verklebten Polymerteilchens (1 "), so dass nach dem Stoß (4) sich die Polymerteilchen (1, 1', 1") einzeln fortbewegen. Durch den Stoß wird eine gleichmäßige Benetzung der Oberflächen erzielt.

Das erfindungsgemäße Verfahren zur Herstellung eines absorbierenden Polymers weist einen ersten Mischvorgang, bei dem eine Vielzahl von absorbierenden Polymerteilchen (1) mit einer Flüssigkeit (2) vermischt werden, und einen zweiten Mischvorgang, bei dem die Flüssigkeit (2) im Inneren der Polymerteilchen (1) homogenisiert wird, auf, wobei, die Polymerteilchen (1) in dem ersten Mischvorgang mit einer Geschwindigkeit gemischt werden, dass die Bewegungsenergie der einzelnen Polymerteilchen (1) im Mittel größer ist als die Haftungsenergie zwischen den einzelnen Polymerteilchen (1), und die Polymerteilchen (1) in dem zweiten Mischvorgang mit einer geringeren Geschwindigkeit als im ersten Mischvorgang durchmischt werden. Durch die unterschiedlichen Geschwindigkeiten wird eine Fluidisierung der Polymerteilchen (1) bewirkt, die eine Verklumpung der Polymerteilchen (1) während des Mischvorgangs vermeidet. Die so hergestellten absorbierenden Polymere bzw. ein Verbund bzw. ein chemisches Produkt, beinhaltend ein derartiges Polymer, zeichnet sich durch ein besonders schnelles Quellverhalten aus.

Die Erfindung wird nun anhand von Testmethoden und nicht limitierenden Beispielen näher erläutert.

**TESTMETHODEN**

BESTIMMUNG DES ABFALLS DES AAP-WERTES

Zur Bestimmung des Abfall des gemäß ERT 442.1-99 bei einem Druck von 0,7 psi bestimmten AAP-Wertes infolge einer mechanischen Behandlung der Polymerteilchen in einer Kugelmühle wurde zunächst der AAP-Wert der Polymerteilchen nach ERT 442.1-99 bei einem Druck von 0,7 psi bestimmt ($AAP_1$). Anschließend wurden diese Polymerteilchen einer Schädigung durch einen mechanischen Stress ausgesetzt. Dazu wurden 10,0 g $\pm$ 0,1 g Superabsorber in einem Porzellanbehälter mit 24 Porzellanzylindern eingewogen und verschlossen. Zwei Porzellanbehälter wurden anschließend auf den Rollen einer Kugelmühle (Jar Mill, US-Stoneware), die mittels eines Foto-Kontakt-Tachometers so eingestellt wurde, dass 95 UpM erreicht wurden, platziert und die Kugelmühle wird gestartet. Die Umdrehungszeit wird mittels einer Stopuhr nach genau 6 Minuten beendet. Anschließend wurde erneut der AAP-Wert der so behandelten Polymerteilchen bei einem Druck von 0,7 psi bestimmt ($AAP_2$). Der Abfall des AAP-Wertes ist definiert als

$$\text{Abfall des AAP-Wertes [\%]} = 100\% - ((AAP_2/AAP_1) \times 100)$$

wobei jeweils der Mittelwert für die $AAP_2$- bzw. $AAP_1$-Werte aus 20 Einzelmessungen für die Bestimmung des Abfalls des AAP-Wertes zugrunde gelegt wurde.

BESTIMMUNG DER AUFNAHMEZEIT (ACQUISITION TIME)

Zur Bestimmung der Aufnahmezeit wurde eine körpergeformte Prüfeinrichtung eingesetzt, deren Querschnitt in Figur 3 gezeigt wird. Diese bestand aus einem körpergeformten halbzylinderförmigen Einsatz (6) mit einer Länge von 20,5 cm mit einer runden Öffnung (7) und einem Einlassrohr (8). Alle Flächen der verwendeten Teile der Prüfeinrichtung sind glatt. Wie sich aus Figur 4 ergibt, die eine Detailansicht der Öffnung (7) darstellt, wies diese Öffnung (7) ein Sieb (9) mit einer Maschenweite von 3 bis 4 mm auf. Der Einsatz (6) wurde in einer ebenfalls im wesentlichen halbkreisförmigen Wanne (10) eingesetzt. Zwischen dem Einsatz (6) und der Wanne (10) wurde in dem Bereich der Öffnung (7) des Einlassrohrs (8) der Prüfkörper (11) auch Composite genannt, eingesetzt. Die genauen Abmessungen des Einsatzes (6), der Öffnung (7) sowie des Einlassrohrs (8) ergeben sich aus Figur 5. Ferner wurde bei der Messung der Aufnahmezeit eine Waage mit einer Messgenauigkeit von 0,01 g sowie Gewichte zur Belastung der Prüfapparatur von einem Kilo $\pm$ 0,5 g bzw. pneumatische Stempel, ein Kurzzeitwecker, ein Rundfilter-Filterpapier 90 mm zur quantitativen Analyse der Firma Schleicher & Schuell, Sorte 589/1, Schwarzbrand sowie eine 0,9 Gew.-%ige Natriumchlorid-Aquadest-Testlösung, die mit 5ml/l Säurefuchsin-Stammlösung eingefärbt wurde. Das zu testende Composite wurde eine Größe 12 cm mal 30 cm zugeschnitten und zentrisch in die körpergeformte Prüfapparatur zwischen den Einsatz (6) und die Wanne (10) gelegt. Der Einsatz (6) wurde mit 9 kg belastet. Es wurden dreimal 80 g der Testlösung durch das Einlassrohr (8) über die Öffnung (7) dem zu testendem Composite in Intervallen von 20 Minuten zugeführt, wobei das 20 Minuten Intervall mit der Zugabe der Testlösung beginnt und die Testlösung auf einmal in das Einlassrohr gegeben wurde. Die Messung der Aufnahmezeit begann mit der Beendigung der Zugabe der Testlösung und endete mit dem vollständigen Versickern der in dem Einlassrohr befindlichen Testlösung. Das vorstehende Messverfahren wurde 3 mal wiederholt und aus den so erhaltenen Werten jeweils der Durchschnitt gebildet.

BESTIMMUNG DES REWET-WERTES

Zur Bestimmung des Rewet-Wertes wurde ein Versuchsaufbau gemäß Figur 6 und 7 verwendet. In eine Petrischale (12) wurde das zu messende Composite (11) als kreisrunder Probkörper mit einem Durchmesser von 9 cm, dessen Gewicht bestimmt wurde, mittig gelegt. Mittels einer Bürette (13) wurden 80 g der zuvor beschriebenen Testlösung zentral auf den Probenkörper in einer Fließgeschwindigkeit von 1,8 - 2,0 ml/s gegeben. Nach Abschluss der Zugabe der Testlösung wurde ein 40,0 g schwerer ebenfalls kreisförmiger Filterpapierstapel (14) mit einem Durchmesser von 9 cm aus Schwarzbrandfilterpapier zur quantitativen Analyse der Firma Schleicher & Schuell Sorte 589/1 gewogen ($F_T$) und vorsichtig auf die Oberseite des Probenkörpers gelegt. Danach wurde der Filterpapierstapel (14) durch Gewicht (15) mit 1.270 g auf ca. 20 g/mm$^2$ für 10 Minuten belastet. Nach Beendigung der Belastung wurde der Filterpapierstapel (14) vorsichtig von dem Probenkörper entfernt und erneut gewogen ($F_F$). Der Rewet-Wert wird bestimmt durch $F_T - F_F$ [g]. Die Messung wird 5 mal wiederholt und der Durchschnittswert gebildet.

**BEISPIELE**

Zur Herstellung eines nachvernetzten absorbierenden Polymers

In 964,515 g einer wässrigen Lösung von Natriumacrylat mit einem Neutralisationsgrad von 70 mol-% (Monomer-Konzentration: 37,7%) werden 1,50 g Polyethylenglycol-300-diacrylat und 1,50 g Polyethylenglycol-750-monoallyletheracrylat als Vernetzer gelöst. Die Monomerenlösung wurde in einem Kunststoff-Polymerisationsgefäß für 30 Minuten mit Stickstoff durchspült, um den den gelösten Sauerstoff zu entfernen. Bei einer Temperatur von 4°C wurde die Polymerisation durch aufeinanderfolgende Zugabe von 0,3 g Natriumperoxidisulfat in 10 g dest. Wasser, 0,1 g 2,2'-Azobis-2-amidinopropandihydrochlorid in 10 g dest. Wasser, 0,07 g 35%ige Wasserstoffperoxidlösung in 10 g dest. Wasser und 0,015 g Ascorbinsäure in 2 g dest. Wasser gestartet. Nachdem die Endtemperatur ca. 100 °C erreicht war, wurde das Gel mit einem Fleischwolf zerkleinert und 2 h bei 150°C in einem Umluftofen getrocknet. Das getrocknete Produkt wurde grob zerstoßen, gemahlen und die Partikel der Größe von 150 - 850 $\mu$m zur weiteren Umsetzung ausgesiebt (Pulver A).

Vergleichsbeispiel 1:

50 g Pulver A wurden unter kräftigem Rühren mit einer Lösung 0,15 g Aluminiumsulfat-18-Hydrat und 0,15 g Wasser und anschließend mit einer Lösung aus 0,3 g Ethylencarbonat und 0,3 g Wasser vermischt und anschließend für 90 Minuten in einem Umluftofen bei 170°C gehalten (Pulver B). Das Pulver B ist durch die in Tabelle 1 aufgeführten Eigenschaften gekennzeichnet.

Tabelle 1

| Eigenschaft von Pulver B | Einheit | Messergebnis |
|---|---|---|
| CRC | g/g | 28,7 |
| AAP (0,7 psi) | g/g | 21,6 |
| Abfall des AAP (0,7 psi) | % | 24 |

Beispiel 1:

200g des nachvernetzten absorbierenden Polymers (Pulver B) wurde in eine Rührtopf eines schnelldrehenden MTI-Mischers (MTI-Mischtechnik, Industrieanlagen GmbH, 322758 Detmold: Typ LM 1,5/5; Baujahr 1995) der (Volumen ca.1l) mit einer Geschwindigkeit der Mischaggregate von 1000 upm rotiert gegeben. 4 ml entionisiertem Wasser erfolgte mit einer Spritze zudosiert. Nach ca. 5 bis 15 Sekunden ist die Flüssigkeit und das absorbierende Polymer homogen vermischt. Nach einer Wartezeit von 15 Minuten (Reifung) erfolgte ein zweiter Rührvorgang mit einem Krups-Drei-Mixer 3000 auf Stufe 1 (langsamste Drehzahl) über 5 Minuten, hierbei wurden die noch aneinander haftenden Teilchen voneinander gelöst und ein fließfähiges, klumpenfreies Material erhalten (Pulver C). Das Pulver C ist durch die in Tabelle 2 aufgeführten Eigenschaften gekennzeichnet.

Tabelle 2

| Eigenschaft von Pulver C | Einheit | Messergebnis |
|---|---|---|
| CRC | g/g | 28,8 |
| AAP (0,7 psi) | g/g | 21,2 |
| Abfall des AAP (0,7 psi) | % | 4,8 |

Herstellung von Composites für Probenkörper

Die Composites wurden mittels einer Mischung von 50 Gew.-% eines absorbierenden Polymers (Pulver B oder C), bezogen auf das Composite, und 47,5 Gew.-% Zellulosefasern Stora Fluff EF semitreated der Stora-Enzo AB Schweden, sowie 2,5 Gew.-% einer Zweikomponentenfaser aus jeweils 50 Gew.-% Polypropylen (PP) bzw. Polyethylen (PE) mit einem PP Kern und einem PE Mantel der Firma Fibervision A/S Dänemark durch ein Airlaid-Verfahren mit einer M&J-Maschine (Breite 40 cm, Arbeitsbreite 36 cm, Betriebseinstellungen: Bandgeschwindigkeit 2 m/min, Fluffeinzug an Hammermühle 3,6 m/min, Polymerdosierung 400 g/min, Zweikomponentenfaser in 10 g Portionen 0,7 mal/min abgeworfen) gebildet, wobei das absorbierende Polymer homogen eingetragen wurde. Composites mit einem Basisgewicht von 870 g/m$^2$ inkl. Tissue (1 Lage, 18 g/m$^2$), einer Dichte von 0,16 g/cm$^3$ wurden als Probenkörper für die nachfolgenden Tests verwendet. Die Ergebnisse der durchgeführten Tests sind in der Tabelle 3 zusammengestellt.

Tabelle 3

| Probe | Aufnahmezeit nach erster Benetzung [s] | Aufnahmezeit nach zweiter Benetzung [s] | Aufnahmezeit nach dritter Benetzung [s] | Rewet [g] |
|---|---|---|---|---|
| Pulver B | 53 | 253 | 475 | 12,55 |
| Pulver C | 45 | 197 | 399 | 10,48 |

Bezugszeichenliste

| | |
|---|---|
| 1 | Polymerteilchen |
| 1', 1" | miteinander verklebt Polymerteilchen |
| 2 | Flüssigkeit |
| 3 | benetztes Polymerteilchen |
| 4 | Geschwindigkeitsrichtung des Polymerteilchen 1 bzw. der Polymerteilchen 1, 1' oder 1" |
| 5 | Haftschicht zwischen zwei Polymerteilchen 1 |
| 6 | Einsatz |
| 7 | Öffnung |
| 8 | Einlassrohr |
| 9 | Sieb |
| 10 | Wanne |
| 11 | Composite |
| 12 | Petrischale |
| 13 | Bürette |
| 14 | Filterpapier |
| 15 | Gewicht |

**Patentansprüche**

1. Verfahren zur Herstellung eines absorbierenden Polymers aufweisend einen ersten Mischvorgang, bei dem eine Vielzahl von absorbierenden Polymerteilchen mit einer Flüssigkeit vermischt werden, und einen zweiten Mischvorgang, bei dem die Flüssigkeit im Inneren der Polymerteilchen verteilt wird, wobei die Polymerteilchen in dem ersten Mischvorgang mit einer Geschwindigkeit gemischt werden, dass die Bewegungsenergie der einzelnen Polymerteilchen im Mittel größer ist als die Haftungsenergie zwischen den einzelnen Polymerteilchen, und die Polymerteilchen in dem zweiten Mischvorgang mit einer geringeren Geschwindigkeit als im ersten Mischvorgang durchmischt werden, wobei die eingesetzten Polymerteilchen einen Innenbereich, einen den Innenbereich umgebenden Aussenbereich sowie einen den Aussenbereich umgebenden Oberflächenbereich aufweisen, wobei der Aussenbereich einen höheren Vernetzungsgrad als der Innenbereich aufweist, wobei die Froude-Zahl beim ersten Mischvorgang zwischen 1,5 und 50 und beim zweiten Mischvorgang zwischen 0,001 und 1 beträgt, und wobei zwischen dem ersten Mischvorgang und dem zweiten Mischvorgang ein Reifevorgang liegt, in dem die Polymerteilchen weniger als in dem ersten und zweiten Mischvorgang gegeneinander bewegt werden.

2. Verfahren nach Anspruch 1, **dadurch gekennzeichnet, dass** die Froude-Zahl beim ersten Mischvorgang zwischen 1,5 und 40, vorzugsweise zwischen 1,7 und 33, und beim zweiten Mischvorgang zwischen 0,01 und 0,2, vorzugsweise zwischen 0,08 und 0,03, beträgt.

3. Verfahren nach einem der vorherigen Ansprüche, **dadurch gekennzeichnet, dass** die mittlere Verweilzeit des ersten Mischvorgangs zwischen 5 und 200 sec, insbesondere zwischen 10 und 100 sec, vorzugsweise zwischen 20 und 60 sec beträgt.

4. Verfahren nach einem der vorherigen Ansprüche, **dadurch gekennzeichnet, dass** zur schonenden Vermischung der statische Druckaufbau während des ersten Mischvorganges kleiner als 0,1 bar, insbesondere kleiner als 0,05 bar, vorzugsweise kleiner als 0,01 bar beträgt.

5. Verfahren nach einem der vorherigen Ansprüche, **dadurch gekennzeichnet, dass** als Flüssigkeit Wasser oder wässerige Lösung zugegeben wird.

6. Verfahren nach Anspruch 5, **dadurch gekennzeichnet, dass** die Flüssigkeit Zusatzstoffe, insbesondere Alkohole, enthält.

7. Verfahren nach einem der vorhergehenden Ansprüche, wobei die Polymerteilchen auf
($\alpha$1) 0,1 bis 99,999 Gew.-% polymerisierten, ethylenisch ungesättigten, säuregruppenhaltigen Monomeren oder deren Salze oder polymerisierten, ethylenisch ungesättigten, einen protonierten oder quarternierten Stickstoff beinhaltenden Monomeren, oder deren Mischungen,
($\alpha$2) 0 bis 70 Gew.-% polymerisierten, ethylenisch ungesättigten, mit ($\alpha$1) copolymerisierbaren Monomeren,
($\alpha$3) 0,001 bis 10 Gew.-% eines oder mehrerer Vernetzer,
($\alpha$4) 0 bis 30 Gew.-% wasserlöslichen Polymeren, sowie ($\alpha$5) 0 bis 20 Gew.-% eines oder mehrerer Hilfsstoffe basieren, wobei die Summe der Gewichtsmengen ($\alpha$1) bis ($\alpha$5) 100 Gew.-% beträgt.

8. Verfahren nach einem der vorherigen Ansprüche, wobei die Polymerteilchen mindestens eine der folgenden Eigenschaften aufweisen:

(A) die maximale Aufnahme von 0.9 Gew.-%er wässriger NaCl-Lösung liegt in einem Bereich von 10 bis 1000 g/g SAP Granulat,
(B) der mit 0.9 Gew.-%er wässriger NaCl-Lösung extrahierbare Anteil beträgt weniger als 30, bezogen auf das SAP Granulat,
(C) die Schüttdichte liegt im Bereich von 300 bis 1000 g/l,
(D) der pH- Wert von 1 g des SAP Granulats in 1 l Wasser liegt im Bereich von 4 bis 10,
(E) der CRC- Wert liegt im Bereich von 10 bis 100 g/g,
(F) der AAP-Wert bei einem Druck von 0,7 psi liegt im Bereich von 10 bis 60 g/g, (G) der AAP-Wert bei einem Druck von 0,3 psi liegt im Bereich von 10 bis 100 g/g.

**Claims**

1.  Process for producing an absorbent polymer, having a first mixing operation in which a multitude of absorbent polymer particles are mixed with a liquid, and a second mixing operation in which the liquid within the polymer particles is distributed, wherein the polymer particles are mixed in the first mixing operation at such a speed that the kinetic energy of the individual polymer particles is greater on average than the adhesion energy between the individual polymer particles, and the polymer particles are mixed in the second mixing operation at a lower speed that in the first mixing operation,
    wherein the polymer particles used have an inner region, an outer region that surrounds the inner region and a surface region that surrounds the outer region, wherein the outer region has a higher level of crosslinking than the inner region,
    wherein the Froude number in the first mixing operation is between 1.5 and 50 and in the second mixing operation is between 0.001 and 1,
    and wherein there is a maturing operation between the first mixing operation and the second mixing operation in which movement of the polymer particles relative to one another less is than in the first and second mixing operations.

2.  Process according to Claim 1, **characterized in that** the Froude number in the first mixing operation is between 1.5 and 40, preferably between 1.7 and 33, and in the second mixing operation is between 0.01 and 0.2, preferably between 0.08 and 0.03.

3.  Process according to either of the preceding claims, **characterized in that** the mean residence time in the first mixing operation is between 5 and 200 sec, especially between 10 and 100 sec, preferably between 20 and 60 sec.

4.  Process according to any of the preceding claims, **characterized in that** gentle mixing is accomplished by virtue of the static pressure buildup during the first mixing operation being less than 0.1 bar, especially less than 0.05 bar, preferably less than 0.01 bar.

5.  Process according to any of the preceding claims, **characterized in that** the liquid added is water or aqueous solution.

6.  Process according to Claim 5, **characterized in that** the liquid comprises additives, especially alcohols.

7.  Process according to any of the preceding claims, wherein the polymer particles are based on
    ($\alpha$1) 0.1% to 99.999% by weight of polymerized ethylenically unsaturated monomers bearing acid groups, or salts thereof, or polymerized ethylenically unsaturated monomers containing a protonated or quaternized nitrogen, or mixtures thereof,
    ($\alpha$2) 0% to 70% by weight of polymerized ethylenically unsaturated monomers copolymerizable with ($\alpha$1),
    ($\alpha$3) 0.001% to 10% by weight of one or more crosslinkers,
    ($\alpha$4) 0% to 30% by weight of water-soluble polymers, and
    ($\alpha$5) 0% to 20% by weight of one or more auxiliaries, where the sum total of the weights ($\alpha$1) to ($\alpha$5) is 100% by weight.

8.  Process according to any of the preceding claims, wherein the polymer particles have at least one of the following properties:

    (A) the maximum absorption of 0.9% by weight aqueous NaCl solution is within a range from 10 to 1000 g/g of SAP granules,
    (B) the fraction extractable with 0.9% by weight aqueous NaCl solution is less than 30, based on the SAP granules,
    (C) the bulk density is in the range from 300 to 1000 g/l,
    (D) the pH of 1 g of the SAP granules in 1 l of water is in the range from 4 to 10,
    (E) the CRC is in the range from 10 to 100 g/g,
    (F) the AAP at a pressure of 0.7 psi is in the range from 10 to 60 g/g,
    (G) the AAP at a pressure of 0.3 psi is in the range from 10 to 100 g/g.

**Revendications**

1.  Procédé pour la fabrication d'un polymère absorbant présentant un premier cycle de mélange, dans lequel une multitude de particules polymères absorbantes sont mélangées avec un liquide, et un deuxième cycle de mélange, dans lequel le liquide à l'intérieur des particules polymères est réparti, les particules polymères étant mélangées dans le premier cycle de mélange à une vitesse telle que l'énergie de mouvement des différentes particules polymères est en moyenne supérieure à l'énergie d'adhérence entre les différentes particules polymères et les particules polymères étant mélangées dans le deuxième cycle de mélange à une vitesse inférieure à celle dans le premier cycle de mélange,

les particules polymères utilisées présentant une zone interne, une zone externe entourant la zone interne ainsi qu'une zone superficielle entourant la zone externe, la zone externe présentant un degré de réticulation supérieur à celui de la zone interne,

le nombre de Froude, lors du premier cycle de mélange, étant situé entre 1,5 et 50 et, lors du deuxième cycle de mélange, entre 0,001 et 1,

et un cycle de maturation étant situé entre le premier cycle de mélange et le deuxième cycle de mélange, dans lequel les particules polymères sont moins déplacées les unes par rapport aux autres que dans le premier et dans le deuxième cycle de mélange.

**2.** Procédé selon la revendication 1, **caractérisé en ce que** le nombre Froude, lors du premier cycle de mélange, est situé entre 1,5 et 40, de préférence entre 1,7 et 33, et, lors du deuxième cycle de mélange, entre 0,01 et 0,2, de préférence entre 0,08 et 0,03.

**3.** Procédé selon l'une quelconque des revendications précédentes, **caractérisé en ce que** le temps de séjour moyen du premier cycle de mélange est situé entre 5 et 200 sec, en particulier entre 10 et 100 sec, de préférence entre 20 et 60 sec.

**4.** Procédé selon l'une quelconque des revendications précédentes, **caractérisé en ce que**, pour un mélange avec ménagement, la mise sous pression statique pendant le premier cycle de mélange est inférieure à 0,1 bar, en particulier inférieure à 0,05 bar, de préférence inférieure à 0,01 bar.

**5.** Procédé selon l'une quelconque des revendications précédentes, **caractérisé en ce qu'**on ajoute, comme liquide, de l'eau ou une solution aqueuse.

**6.** Procédé selon la revendication 5, **caractérisé en ce que** le liquide contient des additifs, en particulier des alcools.

**7.** Procédé selon l'une quelconque des revendications précédentes, les particules polymères étant à base de
($\alpha$1) 0,1 à 99,999% en poids de monomères polymérisés, éthyléniquement insaturés, contenant des groupes acides ou leurs sels ou de monomères polymérisés, éthyléniquement insaturés, contenant un azote protoné ou quaternisé ou leurs mélanges, ($\alpha$2) 0 à 70% en poids de monomères polymérisés, éthyléniquement insaturés, copolymérisables avec ($\alpha$1),
($\alpha$3) 0,001 à 10% en poids d'un ou de plusieurs réticulants,
($\alpha$4) 0 à 30% en poids de polymères solubles dans l'eau, ainsi que
($\alpha$5) 0 à 20% en poids d'un ou de plusieurs adjuvants,
la somme des quantités pondérales ($\alpha$1) à ($\alpha$5) valant 100% en poids.

**8.** Procédé selon l'une quelconque des revendications précédentes, les particules polymères présentant au moins une des propriétés suivantes :

(A) l'absorption maximale d'une solution aqueuse à 0,9% en poids de NaCl se situe dans une plage de 10 à 1000 g/g de granulat de SAP (superabsorbant polymer - polymère superabsorbant),
(B) la proportion pouvant être extraite par la solution aqueuse à 0,9% en poids de NaCl est inférieure à 30, par rapport au granulat de SAP,
(C) la densité apparente se situe dans la plage de 300 à 1000 g/l,
(D) le pH de 1 g de granulat de SAP dans 1 l d'eau se situe dans la plage de 4 à 10,
(E) la valeur CRC (capacité de rétention à la centrifugeuse) se situe dans la plage de 10 à 100 g/g,
(F) la valeur AAP (absorption against pressure - absorption par rapport à la pression) à une pression de 0,7 psi se situe dans la plage de 10 à 60 g/g,
(G) la valeur AAP à une pression de 0,3 psi se situe dans la plage de 10 à 100 g/g.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9849221 A
- WO 9934843 A
- DE 19543366
- DE 19543368
- US 4286082 A
- DE 2706135
- US 4076663 A

- DE 3503458
- DE 4020780
- DE 4244548
- DE 4323001
- DE 4333056
- DE 4418818